# EUROPEAN PATENT APPLICATION

(11) **EP 2 251 049 A2**
(43) Date of publication of application: **17.11.2010**
(21) Application number: 10159880.3
(22) Date of filing: 14.04.2010
(51) Int. Cl.: A61L 27/42, A61L 27/46, A61L 27/56

(54) **Methods and devices for bone attachment**

(30) Priority: 15.04.2009 US 424000
(71) Applicant: DePuy Products, Inc., Warsaw, IN 46581 (US)
(72) Inventor: Tong, Weidong, Warsaw, IN 46580 (US); Salvati, Lawrence, Goshen, IN 46526 (US)
(74) Representative: Belcher, Simon James

(57) **Abstract**

Devices for attachment to bone include substrate having porous structure that includes a collagen-based material and a calcium-phosphate-based material. The collagen can be disposed as collagen fibrils, and hydroxyapatite can be coated on to the fibrils and the porous structure. The porous structure can be three-dimensional, and can promote bone ingrowth thereto. One or more bioactive agents can be coupled to the modified substrate, which can further promote bone growth. The devices can be made by exposing a substrate having porous structure to a collagen-based material, and depositing calcium phosphate-based material on to the collagen based material and the porous network.

## Description

This invention relates to an implant for fixation to bone and to methods of modifying substrates to improve their characteristics for use as portions of a medical implant.

Medical implants for use as replacement structures in patients have become widespread in their application. In particular, orthopaedic implants for replacing joints or other structures have received a great deal of attention commercially and scientifically. Frequently, orthopaedic implants have a porous metallic surface, which is situated adjacent to bone. Long-term mechanical fixation of the implant in lieu of cement or other adhesives can be achieved by the adjacent bone's growth on to the surface of the implant and/or ingrowth into a porous structure on the surface of the implant.

To promote the long-term fixation, early implant fixation is an important factor. Fixation proceeds by bone growth from the host bone toward the implant surface, which can typically begin about three weeks after the implant is inserted into a recipient. Progressive stabilization of the implant is achieved once the new bone bridges the gap between the implant surface and the original adjacent bone surface. Thus, to improve implant fixation, it can be advantageous to develop new methods and materials that can help promote bone attachment to non-cemented implants by, for example, promoting new bone growth.

In one aspect, the invention provides an implant for promoting fixation to bone, comprising:
a substrate having a modified section comprising a porous network;
a collagen-based material distributed in at least a portion of the porous network; and
a calcium-phosphate-based material distributed upon at least a portion of the modified section, the modified section and the calcium-phosphate-based material forming at least a portion of a mineralized collagenous scaffold for promoting fixation to bone.

Preferably, the calcium-phosphate-based material is distributed on the porous network and the collagen-based material.

Preferably, the collagen-based material comprises a porous structure.

Preferably, the porous network comprises a plurality of elements attached to a body of the substrate.

Preferably, the porous network comprises a three-dimensional porous network.

Preferably, the implant includes an extended structure contacting the porous network, the extended structure comprising a porous structure including collagen.

Preferably, the calcium-phosphate-based material comprises apatite.

Preferably, the porous network comprises a metallic material, for example at least one of titanium, chromium, and cobalt.

Preferably, the porous network comprises a textured surface.

Preferably, the collagen-based material comprises type I collagen.

Preferably, the implant includes a bioactive agent coupled to the modified section of the substrate.

Preferably, the bioactive agent comprises at least one of marrow cells, osteoprogenitor cells, pharmacological agents, antibiotics, plasma, platelets, a transforming growth factor, a platelet-derived growth factor, an angiogenic growth factor, a protein related to parathyroid hormone, a cell-attachment modulation peptide, a fibronectin analogue peptide, an antimicrobial agent, an analgesic, and an anti-inflammatory agent.

In another aspect, the invention provides a method of preparing a mineralized collagenous scaffold to promote bone fixation with an implant, comprising:
exposing a collagen-based material to a porous network of a substrate; and
depositing a calcium-phosphate-based material on at least one of the collagen-based material and the porous network to form the mineralized collagenous scaffold of the implant.

Preferably, the step of depositing the collagen-based material to the porous network comprises exposing the at least a portion of the porous network to a collagen-containing mixture, and forming collagen fibrils in the porous network from the mixture.

Preferably, the step of exposing comprises using an acidic-collagen-containing mixture, and forming collagen fibrils comprises exposing the porous network to a less acidic mixture than the acidic-collagen-containing mixture to form collagen fibrils.

Preferably, the step of using the acidic-collagen-containing mixture comprises exposing the porous network to a vacuum before forming the collagen fibrils.

Preferably, the method includes the step of forming a textured surface on at least a portion of the porous network before attaching the collagen-based material to the porous network.

Preferably, the method includes the step of applying a bioactive agent to commingle with collagen-based material.

Preferably, the step of applying the bioactive agent comprises applying a vacuum to drive application of the bioactive agent.

Preferably, the method includes crosslinking the collagen-based material.

Devices provided by the invention can be used in a method of promoting bone fixation of an implant within a subject, comprising:
providing a substrate comprising:
   (i) a modified section forming at least a portion of a surface of the implant, the modified section comprising a porous network,
   (ii) a collagen-based material distributed in at least a portion of the porous network of the modified section of the implant, and
   (iii) a calcium-phosphate-based material distributed upon at least a portion of the modified section; and
      implanting the substrate in proximity to bone of the subject to promote bone fixation of the implant.

The invention provides implants for promoting fixation to bone. The implant includes a substrate with a modified section, which can have a porous network. The porous network can be three-dimensional in nature, and/or include a textured surface. The porous network can comprise a metallic material (e.g., a metallic porous network), which can include one or more of cobalt, chromium, and titanium. Porous networks can be formed as an integral part of the substrate, or can comprise a plurality of elements attached to the substrate's body.

A collagen-based material can be distributed in at least a portion of the porous network. One example of a collagen-based material is type I collagen, though other types and mixtures of collagen can also be utilized. The collagen-based material can comprise fibrils of collagen, which can optionally be crosslinked. In some instances, the collagen-based material itself forms a porous structure. As well, a calcium-phosphate-based material, such as apatite, can be distributed upon at least a portion of the modified section of the substrate. For example, the calcium-phosphate-based material can be distributed on the porous network and/or the collagen-based material. The modified section and the calcium-phosphate-based material can form at least a portion of a mineralized collagenous scaffold for promoting implant fixation to bone.

The implant can also include an extended structure. Such a structure can contact the porous network, and can have a porous structure, which can be oriented to be bone-facing. In some instances, an extended porous structure can comprise collagen, which can help promote bone ingrowth into the implant. One or more bioactive agents can be coupled to the modified section of the substrate and/or the extended structure. Examples of bioactive agents include one or more of marrow cells, osteoprogenitor cells, pharmacological agents, antibiotics, plasma, platelets, a transforming growth factor, a platelet-derived growth factor, an angiogenic growth factor, a protein related to parathyroid hormone, a cell-attachment modulation peptide, a fibronectin analogue peptide, an antimicrobial agent, an analgesic, and an anti-inflammatory agent.

The invention also provides methods for preparing a mineralized collagenous scaffold, which can promote bone fixation within an implant. A collagen-based material can be exposed to a porous network of a substrate. A calcium-phosphate-based material can be deposited on the collagen-based material, the porous network, or both to form the implant's mineralized collagenous scaffold.

The porous network can include metal. It can form a porous metallic network. In some instances, a textured surface is formed on at least a portion of the porous network, e.g., before the network is exposed to other materials. Collagen-based material exposure can be performed by exposing at least a portion of the porous network to a collagen-containing mixture. Collagen fibrils can form from the mixture in the porous network, which can optionally be crosslinked. In some instances, an acidic collagen-containing mixture can be used, which can be in the form of a solution. Exposure (e.g., immersion) of the porous network to a mixture that is less acidic than the collagen-containing mixture can result in collagen fibril formation. A vacuum can be used to facilitate mixture transport in the porous network (e.g., exposing the network to a vacuum to transport the collagen-containing mixture and/or the less acidic mixture through the network). The vacuum technique can also be used to transport the calcium-phosphate-based material when it is disposed in a fluid-containing state.

In some embodiments, one or more bioactive agents can be applied to commingle with the collagen-based material. The bioactive agent(s) can include any one or more to the previously mentioned agents. Vacuum or pressure differential can be used to drive bioactive agent transport.

The invention also provides methods for promoting bone fixation of an implant within a subject. A substrate can be provided that includes a modified section forming at least a portion of the surface of the implant. The modified section can include a porous network (e.g., a metallic porous network). A collagen-based material (e.g., type I collagen-fibrils or any other disposition of collagen described herein) can be distributed in at least a portion of the porous network. A calcium-phosphate-based material can be distributed upon at least a portion of modified section of the implant, as well. The substrate/implant can be implanted in proximity to bone of the subject to promote bone fixation.

Embodiments of the invention are described below with reference to the accompanying drawings, in which:
FIG. 1 is a schematic cross-sectional side view of a modified substrate with a porous structure and collagen-based material disposed therein,
FIG. 2 is a schematic cross-sectional side view of the modified substrate of FIG. 1 placed adjacent to a section of bone,
FIG. 3 is a schematic cross-sectional side view of a modified substrate with a porous structure and having collagen-based material and a bioactive agent located therein,
FIG. 4 is a schematic cross-sectional side view of the modified substrate of FIG. 3 placed adjacent to a section of bone,
FIG. 5 is a schematic cross-sectional side view of a modified substrate with a porous structure extending across a gap to a bone section,
FIG. 6 is a schematic cross-sectional side view of the modified substrate of FIG. 5 loaded with a bioactive agent,
FIG. 7A is a schematic cross-sectional side view of textured beads,
FIG. 7B is a schematic cross-sectional side view of beads immersed in a mixture containing a collagen-based material,
FIG. 7C is a schematic cross-sectional side view of beads with collagen fibrils present in the spaces between the beads,
FIG. 7D is a schematic cross-sectional side view of beads with collagen fibrils present in the spaces between the beads, with a calcium-phosphate-based material adhering to the fibrils and the beads,
FIG. 8 is a schematic cross-sectional side view of an apparatus that can be used to manufacture portions of a modified substrate,
FIG. 9 is a reproduction of a micrograph showing beads which can provide a porous surface structure on an implant, having type I collagen adhering to the beads and forming a bridge between two beads,
FIG. 10A is a reproduction of a micrograph showing beads with microtexturing on their surfaces, and having collagen fibrils on and between the beads,
FIG. 10B is a reproduction of a micrograph having high magnification showing the structure depicted in FIG. 10A,
FIG. 10C is a reproduction of a micrograph showing the microtextured surface of the beads shown in FIGS. 10A and 10B,
FIG. 11 is a bar graph comparing the amount of collagen adhering to an etched disk having a porous surface structure provided by sintered beads, to the amount of collagen adhering to an unetched disk,
FIG. 12A is a reproduction of a micrograph showing beads with nanotexturing on their surfaces, and having collagen fibrils on and between the beads,
FIG. 12B is a reproduction of a micrograph having high magnification showing the structure depicted in FIG. 12A,
FIG. 12C is a reproduction of a micrograph showing the nanotextured surface of the beads shown in FIGS. 12A and 12B,
FIG. 13A is a reproduction of a micrograph showing the beads and collagen fibril sample depicted in FIG. 12A with hydroxyapatite coating portions of the beads and collagen fibrils,
FIG. 13B is a reproduction of a micrograph having high magnification showing the structure depicted in FIG. 13A, and
FIG. 13C is a reproduction of a further magnified micrograph of a section of FIG. 13B showing a collagen fibril attached to a bead with the aid of hydroxyapatite.

Some embodiments of the present invention are directed toward substrates and treatments that can promote bone fixation. In some instances, the substrate has a surface, which can be included with a porous metallic structure, that has a collagen-based material (e.g., type I collagen fibrils) distributed thereon and/or therein. A calcium phosphate-based material, such as a hydroxyapatite, can also be distributed on/in the substrate and/or the collagen-based material. Such a substrate can form a mineralized collagenous scaffold, which can be osteoconductive, e.g., promoting bone fixation to the substrate. Such substrates can be coupled with bioactive agents, such as osteoblasts, to help promote bone growth and attachment when the substrates are used as a portion of a medical implant. Further details and variations regarding such substrates, and methods for producing such materials, are revealed in detail below.

In many embodiments herein, the structures and/or methods described can be utilized with an implant for delivery to a subject. In some embodiments, the implant can be used as a prosthesis for any suitable part of the body, e.g., a component of a joint in the body. Accordingly, implants consistent with some embodiments can find use as a component of an orthopaedic implant, such as, for example, a component of an orthopaedic hip implant, a component of an orthopaedic knee implant, a component of an orthopaedic shoulder implant, a component of an orthopaedic elbow implant, a component of an orthopaedic ankle implant, a component of an orthopaedic finger implant, a component of an orthopaedic spine disk implant, a component of an orthopaedic temporo-mandibular joint (jaw) implant, and other prostheses.

### MODIFIED SUBSTRATES

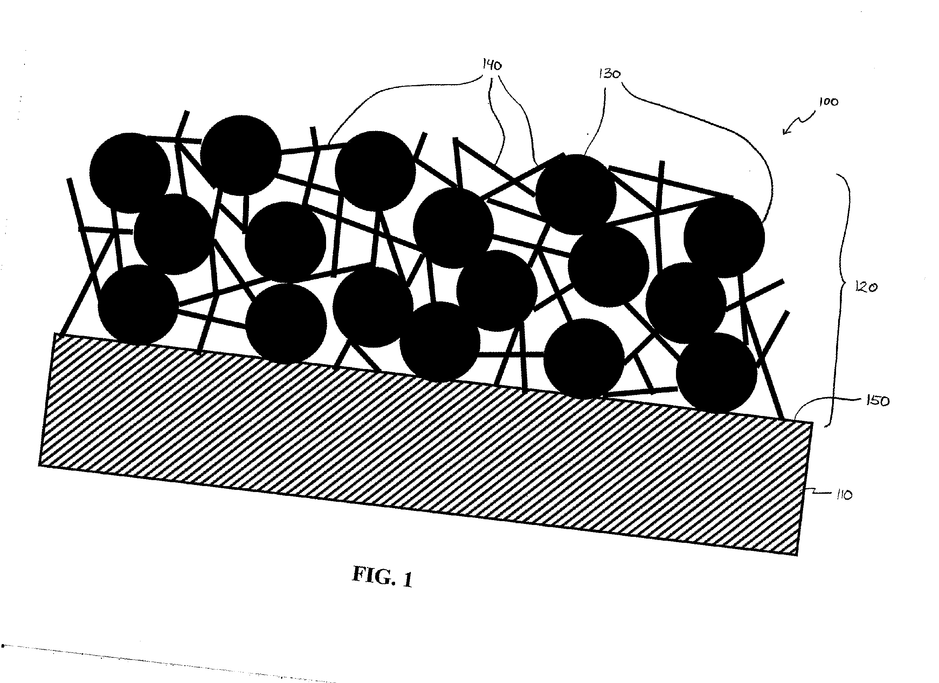

The invention is described with reference to a schematic of an implant, such as a prosthesis, shown by way of example in FIG. 1. A section of the implant includes a modified substrate and its corresponding surface, which can be configured to face a bone section. The modified substrate can also include a porous network. For particular embodiments corresponding with FIG. 1, a substrate 100 includes a body portion 110 with a plurality of elements 130 distributed on the body's surface 150, the elements 130 forming the porous network 120. A collagen-based material can be distributed in the pores of the porous network 120. In instances corresponding with FIG. 1, the collagen-based material can be collagen fibrils, which can be distributed on the elements 130 and/or between the elements, such that porous spaces are still maintained in the network 120. A calcium-phosphate material can also be distributed in the porous network 120.

It is understood that structures consistent with the embodiments enunciated with respect to FIG. 1 only represent some embodiments of the present invention. Indeed, any permutation and combination of the features in FIG. 1 and other figures can be assembled to practise an embodiment consistent with the invention. Other embodiments can include other features, or present modification(s) and/or variation(s) to the features of the embodiments of FIG. 1. Some of the aspects of the embodiments of FIG. 1 are elaborated in further detail herein. Any number of the aspects discussed with respect to an element can be assembled with other elements, or practised individually, consistent with the invention.

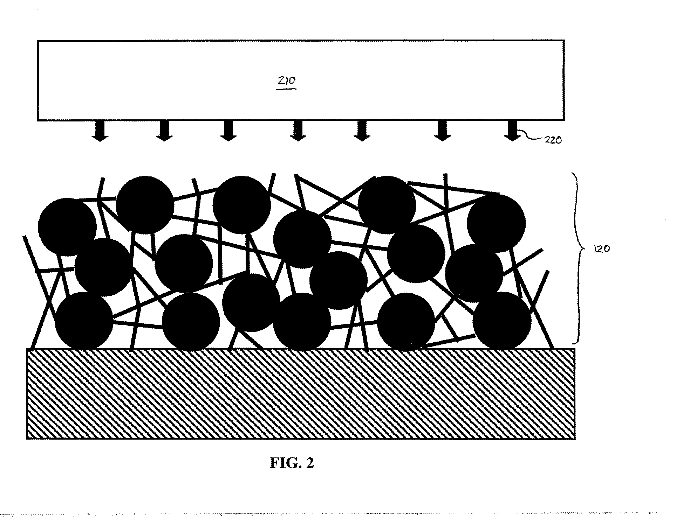

The porous network of a substrate can generally be embodied in a variety of sizes and geometries. In some embodiments, the porous network comprises a three-dimensional network, i.e., the pores of the network can be directed in any dimension. Such networks can have a variety of structures (e.g., open and/or closed cell), and can be formed by a variety of methods, such as etching of a substrate surface with a chemical and/or mechanical technique. In some embodiments, for example as shown in FIGS. 1 and 2, a plurality of elements can be used to form a porous network on a substrate surface. The plurality of elements can be a variety of objects such as particulates, meshes, wires, other elemental structures, and combinations of such elements. In some embodiments, the elements are sized such that at least one dimension of the element has an average size in the range of about 0.1 µm to about 10 mm. For example, particulates, such as beads or irregularly shaped particulates, can be utilized with an average effective diameter of about 250 µm. The average effective diameter of an irregularly shaped particle can be determined using known methods yielding a value independent of determining the precise size of each particulate (e.g., adsorption isotherms). In other embodiments, the elements are arranged so that the porous network has a depth of at least about two times the average smallest dimension of the elements comprising the network. For example, beads can be distributed on a substrate to form a porous surface in which the depth of the beads is at least about 3 times the average bead diameter.

The composition of the solid portion of the porous network and/or the substrate can be anything suitably consistent with the invention. Materials such as metals, ceramics, polymers, and other materials, including composites of materials, can be utilized. In some embodiments, the solid portions of a porous network can be metallic. In general, the term "metallic" is used to describe an object having at least some qualities of bulk metals. Accordingly, metallic porous networks and metallic substrates exhibit at least one property similar to that of bulk metals. For example, non-metallic materials that have trace contaminants of metal impurities can be excluded from a description of metallic materials. Exemplary metallic materials include those suitable for medical implantation within a subject, such as titanium-based materials, titanium-based alloys, and chromium-based alloys (e.g., a cobalt-chromium alloy or a cobalt-chromium-molybdenum alloy). One particular example includes a porous surface structure provided by metallic beads, for example of a cobalt-chromium alloy, as on products sold by DePuy Orthopaedics Inc under the trade mark Porocoat. The porous network can also be integrally formed with the substrate as in products sold by Zimmer Inc under the trade mark Trabecular Metal.

In some embodiments, the substrate and/or the surfaces of the porous network can be treated to enhance the binding of one or more components to the surface. For instance, a treatment can act to make a surface more hydrophilic, which can potentially aid in collagen distribution and/or attachment. For example, plasma treatment or treatment with an acid/base solution and/or surfactants can alter the hydrophilicity of a surface. In another instance, a surface can be textured to increase its relative surface area compared with a smooth surface. A roughened surface can help enhance collagen-based material adhesion thereto. Roughening can be achieved using any number of techniques, including those known to one skilled in the art such as chemical etching, grit blasting, and other chemical and/or mechanical techniques. Some examples of chemical etching techniques for chromium-containing alloys, among others, are disclosed in US-7368065. Nanoetching on chromium containing alloys can be performed using a variety of techniques, such as those disclosed in the application filed with the present application entitled "Nanotextured Cobalt-Chromium Alloy Articles having High Wettability and Method of Producing Same", which claims priority from US patent application no. 61/169365.

The texturing can have a variety of geometries and sizes depending upon the technique used. The "size" of a textured surface refers to a characteristic length scale indicative of the size features of the textures as understood by those skilled in the art. In some circumstances, grit blasting can be used to provide texture on a macro-scale, e.g., on a scale of about hundreds of microns or more. In some embodiments the texturing has a size scale from about 1 nm to about 500 µm. In some instances the texture can be characterized as microtexturing, i.e., size from about 1 µm to about 500 µm, or nanotexturing, i.e., size from about 1 nm to about 1 µm, or a combination of both.

Collagen-based materials, as utilized in some embodiments of the present invention, can include a variety of substances that have collagen in some form. Various types of collagen can be used, though some embodiments utilize types in which the collagen is structured as fibrils (e.g., type I collagen). Collagen-based materials can be naturally occurring fibrils/fibres (e.g., from an extracellular matrix), reconstituted fibrils from solution or other chemical source, or both. In some instances, a collagen-based material can include an agent to help maintain the integrity of a structure (e.g., a fibril). For instance, the collagen-based material can include a crosslinking agent (e.g., gluderaldehyde or glutaraldehyde).

Collagen-based materials can be distributed in a porous matrix in a variety of manners. For instance, the collagen-based material can substantially penetrate the entire porous space, or only a particular section (e.g., penetrating to a selected depth). In some embodiments, the collagen-based material is disposed as fibrils distributed in the porous space. The fibrils can also be attached to the solid structure that can form the frame of the pore spacing (e.g., the fibrils can contact elements, such as beads, that form the porous structure). Such attachment can be by the affinity of the collagen-based material to the solid, or through the use of some intermediary means (e.g., the use of the calcium-phosphate material, as described in more detail herein). In many embodiments, the fibrils can be distributed in the porous space such that substantial pore space remains to allow impregnation with other materials.

Calcium-phosphate-based materials used in some embodiments can include a variety of forms of calcium-phosphate suitable for implantation within a living being. In some embodiments, the calcium-phosphate-based material can include apatite. For instance, the apatite can include compounds of the formula: Ca₅(PO₄)₃ (OH, F, Cl or CO₃. Other non-limiting examples of other suitable biocompatible calcium-phosphate-based materials include: hydroxyapatite, alpha-tricalcium phosphate, beta-tricalcium phosphate, brushite and other calcium hydrogenated-phosphates, polymorphs of calcium phosphate, and combinations of such materials. In accordance with some embodiments, the calcium-phosphate based material includes hydroxyapatite. In some embodiments, the calcium-phosphate-based material is distributed in the pore space of a porous matrix. The calcium-phosphate-based material can adhere to the solid structure of the porous space (e.g., the elements forming a pore space), the substrate, the collagen-based material, or any combination of the three. Like the collagen-based material, the calcium-phosphate material can occupy the pore space in a manner to allow migration of other materials into the pore space, in some embodiments. In some embodiments, the calcium-phosphate material can act to aid adhesion between the collagen-based material (e.g., a collagen fibril) and the porous structure. For example, hydroxyapatite can be coated onto a collagen fibril and the solid porous structure to provide further strength of attachment between a fibril and the porous solid structure.

In some embodiments, an implant having a porous network (e.g., having a three-dimensional porous structure) comprising the collagen-based material and the calcium-phosphate-based material can improve the attachment of bone to the implant without the use of cement or other adhesive. Such a feature can aid the assimilation of the implant into a subject's body. For instance, the porous space can allow bone 210 adjacent to the network 120 to migrate toward 220 and into the network to help stabilize the implant as depicted in FIG. 2. In particular, the combination of the collagen, calcium-phosphate, and porous structure can provide an environment that promotes bone in-growth into the pore spacing, and/or can enhance osteocyte attachment thereto.

In contradistinction, implants that only utilize type I collagen on metallic surfaces have been found by some researchers not to exhibit any significant enhancement of bone ingrowth (see Svehla et al, "No Effect of a Type I Collagen Gel Coating in Uncemented Implant Fixation", J Biomed Mater Res B Appl Biomater. 2005 Jul, 74(1), 423-28; and Becker et al, "Proliferation and Differentiation of Rat Calvarial Osteoblasts on Type I Collagen-Coated Titanium Alloy", J Biomed Mater Res. 2002 Mar 5, 59(3), 516-27). Plasma-sprayed hydroxyapatite on implant surfaces, without the presence of a collagen-based material, has been shown by some researchers to also exhibit problems. For instance, debris and entrapped particles can be generated by the plasma spraying, which can then detrimentally reside in the recipient of the implant. Plasma-spraying can also tend to damage an upper coating of beads, when such are used to make a porous structure, thereby potentially affecting the implant surface that would meet bone. Also, the tendency for the hydroxyapatite to delaminate due to the relatively weak adhesion between a metallic implant surface and the hydroxyapatite coating is problematic.

In some embodiments, a porous structure comprised of a collagen-based material and a calcium-phosphate-based material can be selectively loaded with other materials such as bioactive agents. Bioactive agents are generally materials which can promote a selected biological response in conjunction with the modified substrate when implanted in a subject. In some instances, the bioactive agents are selected to promote bone in-growth into the porous structure. Non-limiting examples of bioactive agents include marrow cells, osteoprogenitor cells, pharmacological agents, antibiotics, plasma, platelets, a transforming growth factor, a platelet-derived growth factor, an angiogenic growth factor, a protein related to parathyroid hormone, a cell-attachment modulation peptide, a fibronectin analogue peptide, an antimicrobial agent, an analgesic, and an anti-inflammatory agent. Such bioactive agents can be inserted into the porous structure during the manufacturing of the substrate, or just before implantation into a subject (e.g., when using live cells in the porous space).

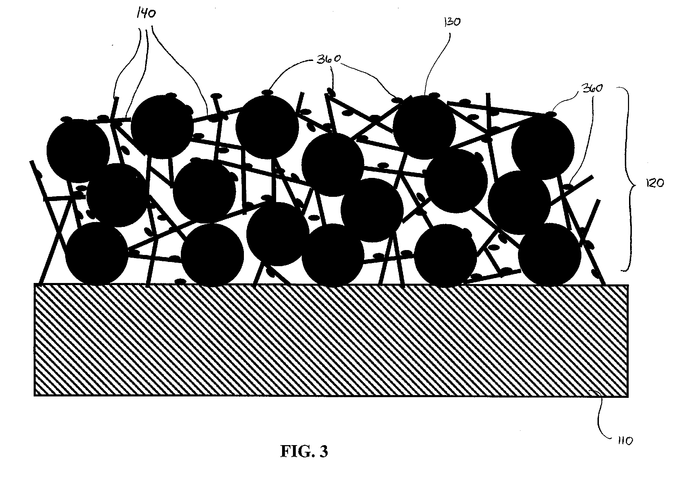

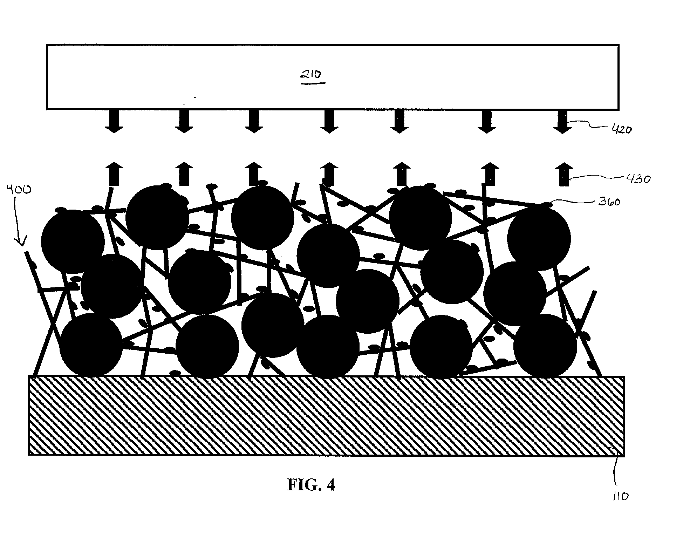

The use of bioactive agents are discussed with reference to FIGS. 3 and 4. As shown in FIG. 3, bioactive agents 360, e.g., aspirated marrow cells, can be loaded into the porous network, where the agents 360 can optionally contact and/or adhere to any structure within the network 120. Without necessarily being bound by any particular theory, when the bioactive agents are marrow cells and/or osteoprogenitor cells, the modified substrate can enhance bone in-growth and/or implant fixation, e.g., by promoting bone growth in two directions. As shown in FIG. 4, an adjacent bone segment 210 can grow toward 420 the modified substrate 400, while the osteoprogenitor cells 360 in the modified substrate 400 can potentially proliferate osteocytes toward 430 the bone segment 210. Alternatively, or in addition, the presence of the osteoprogenitor cells 360 or other bioactive agent(s) in the modified substrate 400 can help promote bone fixation as cells proliferate into the porous space from the bone side 210 by providing an environment conducive to cell proliferation and fixation.

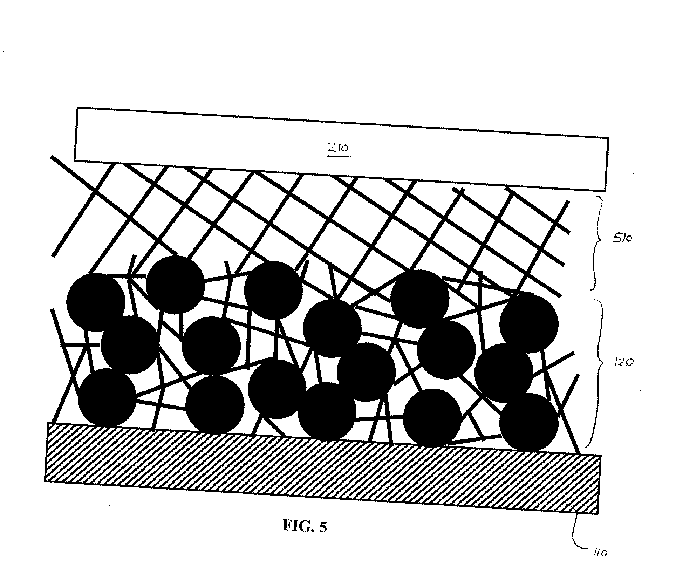

In some embodiments, the modified substrate can include an extended structure that contacts the porous network of a modified substrate. Such an extended structure typically has a porous structure comprising collagen (e.g., having collagen-based material similar to that used in the substrate's modified substrate). The extended porous structure can be osteoconductive, which can aid in bone ingrowth. Some exemplary embodiments can be represented by the schematic of FIG. 5. As depicted, a porous structure comprising collagen-based material 510 can extend from the porous network 120 formed with the collagen-based material, beads, and calcium-phosphate-based material. In some instances, the porous structure 510 is configured to be bone-facing when the implant is inserted into a subject. The structure 510 can be comprised of collagen-fibrils, and/or can form a three-dimensional porous structure. A calcium-phosphate based material can be optionally incorporated into and/or onto the porous structure 510, which can help provide an environment that is osteoconductive. The thickness of the extended porous structure 510 can be selected to span at least a portion of the entirety of a gap space between an implant and a bone surface 210. In some instances, the thickness can be at least about 100 microns or at least about 0.5 mm. The thickness can be not more than about 1 cm, or not more than about 5 mm.

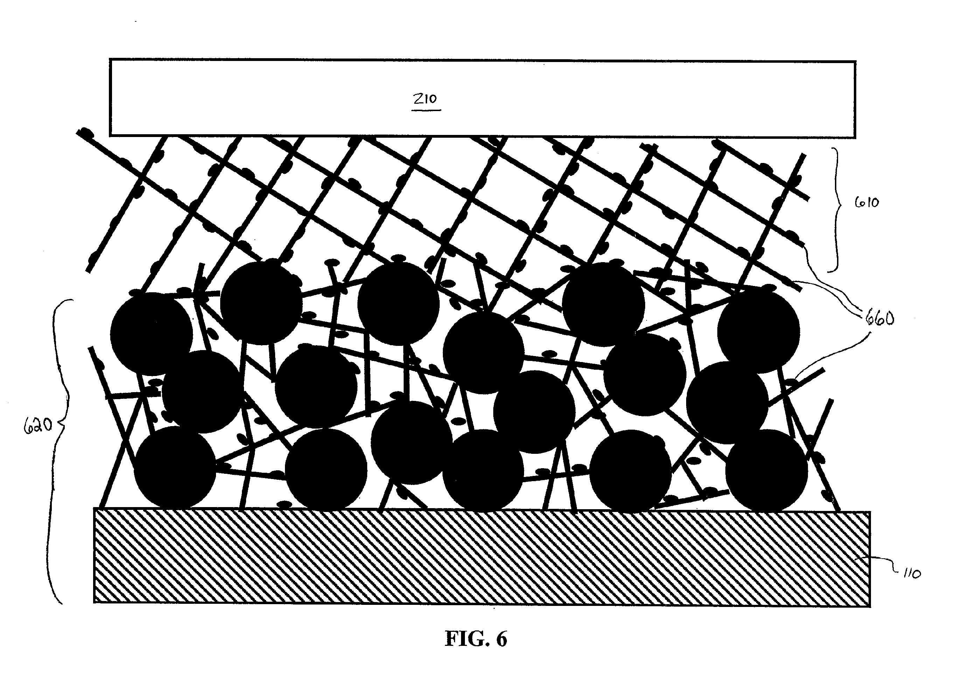

The extended porous structure 510 can help promote tissue growth between a bone 210 and the implant structure 120. For instance the extended structure 510 provides a scaffold that creates an environment favourable for bone ingrowth. The extended porous structure 510 can also act as a bridge between the implant structure 120 and the bone 210 to compensate for potential surgical inaccuracies in fitting of an implant, which can result from, for example, the elasticity of the biological matrix. As shown in FIG. 6, one or more bioactive agents 660 can be added to the porous extension structure 610, which can further promote ingrowth toward the implant. For example, when marrow cells, which can further include other marrow and/or bone growth promoting components (e.g., platelet rich plasma from the implant subject), are seeded into the structure 610, bone growth can be augmented to form in more than one direction, i.e., more than just proceeding from the bone.

In some instances, the extended structure is integrally connected with the porous network of the substrate. For example, with respect to FIG. 6, the extended structure 610 and the remainder of the implant 620 can be connected as an integral porous matrix (e.g., the extended structure is a collagen network that extends from the collagen in the porous metal network of a substrate). In other instances, the extended structure can be a separate structure from the porous network of the substrate. For example, with respect to FIG. 6, the extended structure 610 can be configured as a separate piece from the remainder of the implant 620. Thus, the extended portion 610 could be loaded with one or more bioactive agents just before an implantation procedure (or pre-manufactured beforehand), and inserted between the remainder implant portion 620 and a bone section 210.

### METHODS OF MANUFACTURING MODIFIED SUBSTRATES

The invention provides methods of preparing a material which can promote bone fixation. The material, which can be disposed on an implant surface, can comprise a modified substrate, for example a mineralized collagenous scaffold. In some embodiments, a collagen-based material is deposited in a porous network of a substrate. A calcium-phosphate-based material can be deposited on to the collagen-based material, the surfaces of the porous network, or both to form the scaffold. In many embodiments, the formed scaffold can be consistent with various other embodiments of a modified substrate described in the present application, though other embodiments need not conform to such materials.

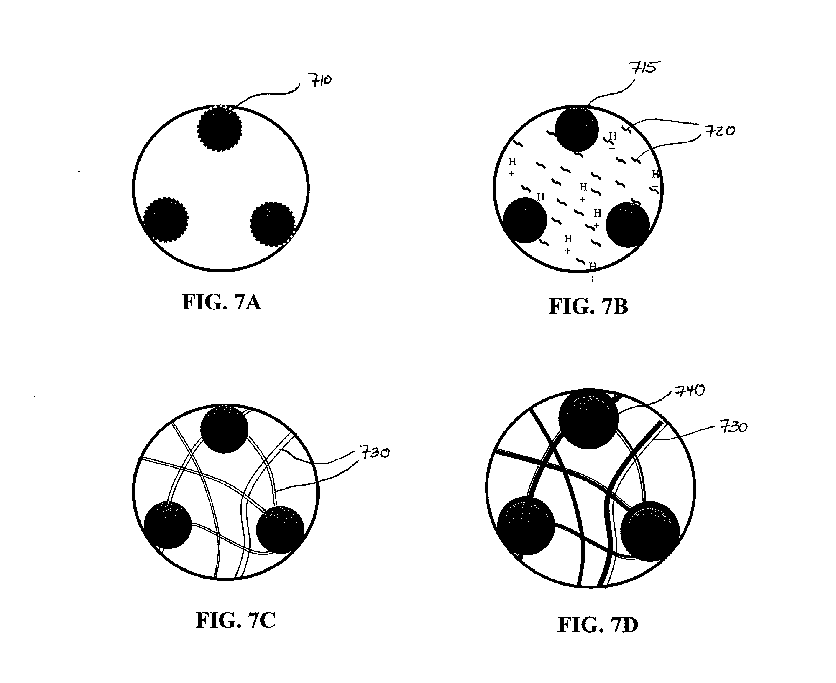

The formation of such materials is described by way of example with reference to the depictions in FIGS. 7A-7D. A plurality of elements, such as the textured beads 710 shown in FIG. 7A, can be used to form the porous network of a substrate. The elements can have a variety of shapes and sizes, as previously described herein, and can be deposited on a surface to form the porous network (e.g., a three-dimensional porous network) using a variety of techniques including those understood by one skilled in the art. As well, other techniques can also be utilized to form the porous network (e.g., a metallic porous network), such as etching, chemical and/or mechanical, of an appropriate substrate to cause removal of material from a surface resulting in a porous structure the desired characteristics.

Deposition of the collagen-based material in a porous network can be performed using many techniques. In some embodiments, the porous network can be exposed to a collagen-containing mixture, where the mixture can then form collagen fibrils within the network. For example, dissolved collagen molecules (e.g., type I having a size in the nanometre range 720) can be disposed in an acidic solution to form a mixture which is exposed to the network formed by elements 715, as shown in FIG. 7B. The mixture can optionally include some bits of collagen-fibrils (e.g., fibrils derived from a bovine source and having sizes in the range of about 1 µm to about 100 µm (micron to tens of microns)) to promote growth of fibrils later in the process. In other embodiments, the collagen-containing solution can have as its main collagen-constituent pieces of collagen fibrils having a size of a few micrometres or larger.

Exposure of the mixture can be performed in a variety of manners, for example by immersing the porous network in the mixture. In some embodiments, a vacuum can be applied to the substrate, which can remove air in the porous space to promote penetration of the mixture. In other embodiments, a pressure source can be applied to drive fluids by providing a pressure higher than the ambient pressure which the porous network is subjected to. An example of a suitable pressure sources is a gas source but other sources might be used.

Collagen material can then be formed from solution (e.g., collagen fibrils formed) in the porous network by raising the pH, i.e., lowering the acidity, of the environment in the porous network to cause collagen to precipitate out into the network as shown in FIG. 7C. The conditions for solubilising a collagen material in solution, and precipitating the formation of fibrils, include those known to one skilled in the art. For instance, collagen can be disposed in a solution having a pH lower than about 2 to keep the collagen in solution. The pH of the solution can then be raised, e.g., to a value greater than about 5, to cause fibril formation. In a particular example, the collagen-loaded porous network can be exposed to a neutral buffered solution at a physiologic temperature (e.g., 37°C) to cause collagen fibrils 730 to self-assemble in the porous network.

In some instances, it can be beneficial to enhance the integrity of collagen-based material that is reconstituted from a mixture (e.g., the fibril network). This can be achieved using any number of agents such as crosslinking agents (e.g., glutaraldehyde and/or gluderaldehyde). The agent, which can be dispersed in another fluid media, can be exposed to the collagen network (e.g., immersing the collagen network in the agent-containing media). The agent can penetrate the fibril network, and interact in a manner to crosslink the collagen molecules and enhance the integrity of the collagen network. When a collagen-containing solution utilizes a substantial amount of micron-sized collagen pieces (e.g., extracted from a bovine source), less agent can be utilized compared with forming a collagen fibril from dissolved collagen molecules, or the use of agent can be avoided entirely in some instances. In some embodiments, a porous network can be exposed to a solution that contains collagen molecules, and calcium and phosphate based materials in solution. A change of conditions can result in collagen-fibril formation and calcium-phosphate-based material deposition thereon in essentially one step. For example, the solution can be in an acidic-environment when the components are solubilised therein. Raising the pH (e.g., lowering the acidity) can then result in fibril formation and calcium-phosphate based deposition.

Deposition of the calcium-phosphate-based material can be performed in a variety of manners. In some embodiments, this can be performed by exposing the porous network with the collagen-based-material to a calcium-phosphate solution, which can be in a metastable state for example. This can cause the deposition of a calcium-phosphate-based solid (e.g., hydroxyapatite 740) in the porous network as shown in FIG. 7D. Some examples of calcium-phosphate-based material deposition techniques are disclosed in US-6569489. Other potential calcium-phosphate-based material forming techniques are disclosed in US-6069295, US-6146686 and US-6344061.

Other chemical or deposition techniques can also be used to deposit the calcium-phosphate-based material and/or collagen-based material. For instance, electrochemical methods can be utilized for deposition. In some embodiments, a potential can be applied between an electrode and a porous structure (e.g., metallic), where both structures contact a solution capable of conducting electricity. The potential can drive deposition of the calcium-phosphate-based material from the solution onto the porous structure. In some embodiments, electrochemical deposition can be utilized to deposit a calcium-phosphate-based material and a collagen-based material substantially simultaneously. Other variations of this technique can also be applied.

After formation of the modified substrate, the product can be freeze-dried to remove excess water and to maintain the state of the material until use.

In some embodiments, one or more bioactive agents can be used to commingle with the collagen-based material and/or calcium-phosphate-based material, the types of bioactive agents being described elsewhere in the present application. A variety of techniques can be utilized to apply the bioactive agents, which can depend upon when the agent is to be placed in the modified substrate. For instance, some bioactive agents can be integrated with the modified substrate during the substrate's manufacturing. In one example, a bioactive agent, such as bone morphogenic protein or other agent capable of being easily stored with the modified substrate, can be dissolved in an acidic solution with dissolved collagen. Precipitation of the collagen, e.g., upon exposure to a higher pH, can also cause commingling of the bioactive agent therewith. In another example, the bioactive agent can be loaded with the depositing of the calcium-phosphate-based material into the porous space. In yet another example, the bioactive agent can be loaded after the collagen-based material and the calcium-phosphate-based material are loaded (e.g., by immersion of the modified substrate in a bioactive agent containing solution). Other materials can be loaded with the bioactive agent, or sequentially thereafter, to promote adhesion of the bioactive agent with the matrix.

In other embodiments, one or more bioactive agents can be loaded into the modified substrate at a time near the use of the substrate, e.g., during an operating procedure for implanting the substrate or near the point of care. Examples of such bioactive agents include agents that are perishable such as bone marrow aspirate or cells such as osteoprogenitor cells, or platelet rich plasma. It is understood, however, that non-perishable agents or agents with substantial longevity can also be loaded at this stage if desired. Various mechanisms can also be utilized to load bioactive agents into the matrix including the use of a vacuum or a wick patch. A vacuum oriented to draw fluid holding the bioactive agent, e.g., bone marrow aspirate, through a porous space, for example, can deposit bioactive agent into the space. A wick patch is a substrate that has an affinity for a fluid to be drawn through the substrate. For example, bone marrow aspirate can be drawn through the substrate by contacting a wick patch with one section of the porous space and a source of the bone marrow aspirate with another section. The tendency of the wick patch to absorb fluid will drive fluid flow through the porous network by capillary action, with the flow drawing bone marrow aspirate from the source to into the porous network. Clearly, other fluids can be utilized including fluids with particulates or other solids or solutes distributed therein.

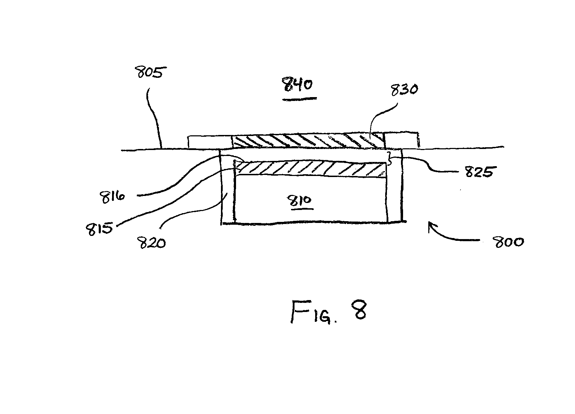

Examples of methods for manufacturing modified substrates are described with reference to the schematic shown in FIG. 8. A fluid-holding vessel 800 can be constructed to promote deposition of a collagen-based material and/or a calcium-phosphate-based material. For instance, an implant 810 having a porous matrix 815 can be inserted into the vessel 800. The vessel 800 can be configured such that a small gap 815 exists between the top of the porous structure 816 and the top of the vessel 805. A semi-permeable membrane 830 can be used as part of a seal over the top of the vessel.

In this configuration, the vessel 800 can be used to deposit materials in the porous matrix 815. For instance, a collagen-containing mixture having dissolved collagen molecules and/or small collagen pieces, can be exposed to the porous portion 815 of an implant, the mixture being acidic. The collagen-containing mixture-entrained implant can be placed in the fluid holding area 820 along with a fluid having a neutral pH. By subjecting fluid area 820 to a physiological temperature, collagen fibril formation can be induced. Fluid can be withdrawn from the fluid holding area 820 by imposing an appropriate pressure differential between the holding area 820 and an opposite side 840 of the membrane 830 (e.g., subjecting environment 840 to a vacuum). The membrane 830 can be substantially impermeable to the collagen-based material (e.g., having a molecular weight cut-off of about 10 kDa compared with the molecular weight of collagen molecules of about 200 kDa). Accordingly, collagen fibrils can be formed in the porous matrix 815. Moreover, the collagen fibrils can also form in the gap region 825, which can form the extended portion of the porous matrix, consistent with some embodiments herein. The gap distance can be specifically chosen to have a distance corresponding with the thickness of the extended portion desired.

The vessel can be used subsequently to perform other depositions. For instance, once the collagen fibrils have been formed, fluid containing a calcium-phosphate based material can be placed into the fluid containment space 820. By drawing fluid through the semi-permeable membrane 830, the calcium-phosphate material can be drawn into the porous region 815 and the extended collagen network. This process can be repeated using fluid containing one or more bioactive agents to deposit such agents as well.

Several variations regarding the vessel 800 and its use are possible. For example, the gap 825 can be substantially eliminated to form a modified substrate without an extended region, following the procedures discussed above. As well, fluid can be driven by placing a pressure source in communication with the fluid holding space 820 to provide a higher pressure therein relative to the external space 840. Vessels can also not utilize a fluid driving force, relying on equilibrium to drive a reaction (e.g., forming collagen fibrils in the pore space). In another variation, the membrane 830 can be replaced with a wick patch to drive fluid flow using a capillary effect, as described consistent with other embodiments herein.

### EXAMPLES

### Example 1: Unetched Beads and Type I Collagen

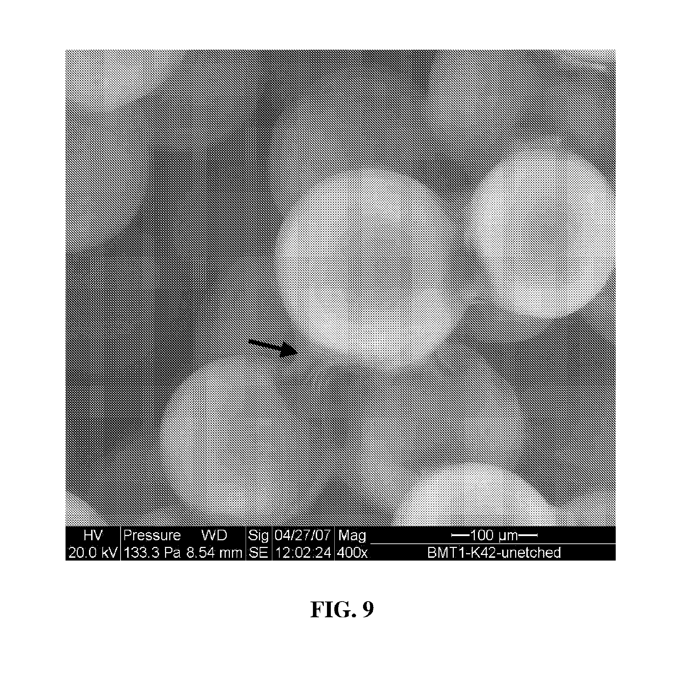

Beads made of a cobalt-chromium-molybdenum alloy, and having a diameter of about 200 µm ere exposed, as received, to a solution having type I collagen; the beads did not have an etched surface. 50 µl of type I collagen (6.4 mg.ml⁻¹, pH 2, supplied by Inamed Corporation under the trade mark Nutragen) was added onto the surface of the beads in multiple droplets to maximize the surface coverage. The droplets were exposed to the beads for 5 minutes so that the collagen was absorbed. The beads were subsequently soaked in 50 ml PBS (100mM Na₂HPO₄, 9% NaCl, pH 6.8 at 25°C) solution at 37°C for 24 hours, followed by a rinse and frozen at -80 °C for 2 hours. The frozen beads were transferred to a bulk tray dryer and lyophilized in a freeze dry system overnight. The collagen formed connections between the beads as shown in FIG. 9.

### Example 2: Microetched Beads and Type I Collagen

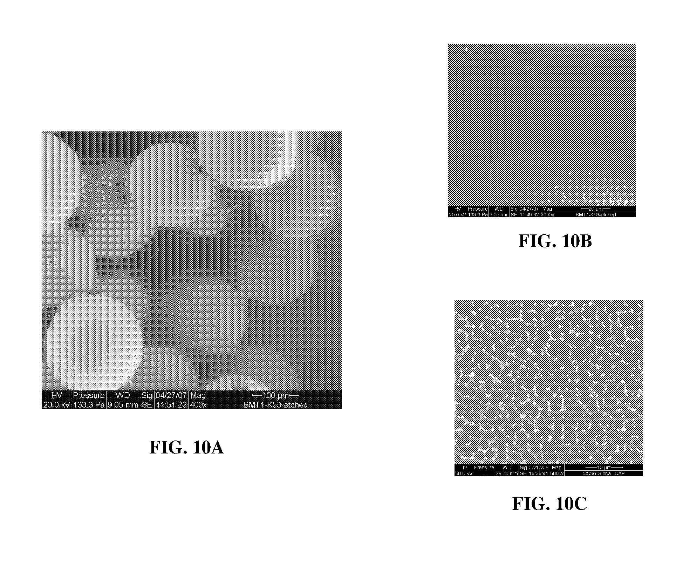

Beads made of a cobalt-chromium-molybdenum alloy were distributed on a cobalt-chromium-molybdenum disk (diameter about 19 mm (0.75 inch); thickness about 0.08 mm (0.003 inch)). The assembly was etched in 6.4N HCl and 0.15 M (NH₄)₂S₂O₈ for 1 hour. The surface exhibited microtexturing, as shown in FIG. 10C. The pores have approximate sizes on the scale of about a micron. Next, 50 µl of type I collagen (6.4 mg.ml⁻¹, pH 2, supplied by Inamed Corporation under the trade mark Nutragen) was added onto the microetched bead-coated disk in multiple droplets to maximize the surface coverage. The droplets were exposed to the bead-coated disk for 5 minutes so that the collagen was absorbed in the disk. The disk was subsequently soaked in 50 ml PBS (100mM Na₂HPO₄, 9% NaCl, pH 6.8 at 25°C) solution at 37 °C for 24 hours. The bead-coated disk was rinsed and frozen at -80 °C for 2 hours. The frozen beads were transferred to a bulk tray dryer and lyophilized in a freeze dry system overnight. The collagen (organic) matrix was successfully integrated with the metal surface and formed a three-dimensional network in between the beads, as depicted by the micrographs in FIGS. 10A and 10B. In particular, FIG. 10B shows the adhesion between the collagen fibrils and the etched surface.

### Example 3: Collagen Adhesion to Bead-Coated Surfaces

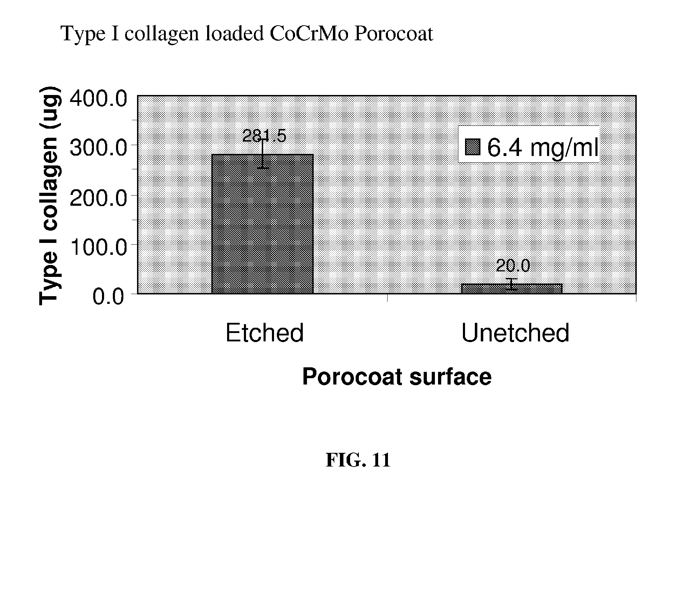

Bead-coated disks (diameter about 19 mm (0.75 inch); thickness about 0.08 mm (0.003 inch)) were either acid etched or used non-etched. 70 µl of type I collagen (6.4 mg.ml⁻¹, pH 2, supplied by Inamed Corporation under the trade mark Nutragen) was added on to the bead-coated disk in multiple droplets to maximize the surface coverage. The droplets were exposed to the bead-coated disk surfaces for 5 minutes so that the collagen was absorbed in the disk. Excessive collagen solution on the disk surface was removed by gently pressing a filter paper on the top of the porous surface that is provided by the beads. The weight gain of the bead-coated disks, etched and unetched, were measured by the weight difference before adding collagen solution and after removing excessive collagen solution on the porous disk surface. As shown in FIG. 11, the etched surfaces retained about fourteen times more collagen than the unetched surfaces.

### Example 4: Nanoetched Beads and Type I Collagen

### Beads were nanoetched using the following technique: Each of the 19 mm

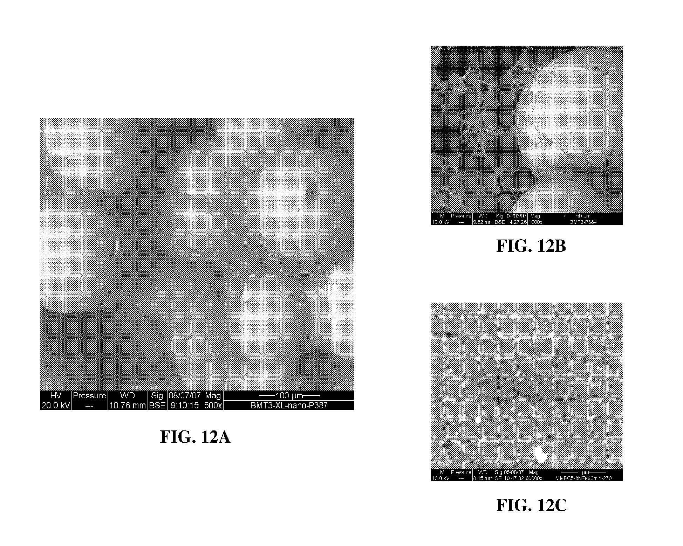

(0.75 inch) diameter bead-coated disks were fully immersed in 50 ml 8N HCl for 24 hours at room temperature. FIG. 12C presents a micrograph of the porous surface, showing that the pores have a size on the order of about 10 nm. The nanoetched beads were coated with collagen using the following technique. 50 µl of type I collagen (6.4 mg.ml⁻¹, pH 2, supplied by Inamed Corporation under the trade mark Nutragen) was added to the nanoetched beads in multiple droplets to maximize the surface coverage. The droplets were exposed to the beads for 5 minutes so that the collagen was absorbed in the beads. The beads were subsequently soaked in 50 ml PBS (100mM Na2HPO₄, 90% NaCl, pH 6.8 at 25°C) solution at 37°C for 24 hours. The beads were rinsed and frozen at -80°C for 2 hours. The frozen beads were transferred to a bulk tray dryer and lyophilized in a freeze dry system overnight. The formed three-dimensional structure is shown in FIGS. 12A and 12B.

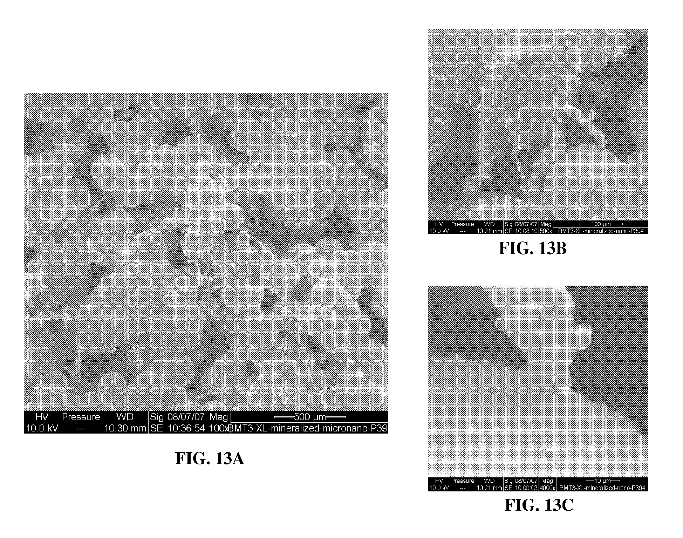

The collagen-loaded porous matrix was mineralized with hydroxyapatite using the following technique. A. mineralization solution was prepared by mixing the following chemicals at the specified concentrations: NaCl (150.2 mM), K₂HPO₄ (2.4 mM), MgCl₂ (2.5 mM), CaCl₂ (6 mM), and (HOCH₂)₃CNH₂ (tris-buffer, 17.5 mM). 18 ml of 1N HCl was added into the final 11 volume solution followed by adding NaHCO₃ (8 mM). The temperature during mineralization was maintained at 37±1°C. Collagen loaded bead-coated disks were incubated in the mineralization solution for more than 12 hours but less than 24 hours in a sealed container. The mineralized final products are fully rinsed in water several times and dried in air. Various magnifications of micrographs showing hydroxyapatite-coated fibrils and beads are presented in FIGS. 13A to 13C. As shown by the micrograph of FIG. 13C, the hydroxyapatite can be used to aid in stabilizing a collagen fibril to the bead surface.

## Claims

1. An implant for promoting fixation to bone, comprising:
a substrate having a modified section comprising a porous network;
a collagen-based material distributed in at least a portion of the porous network;
and
a calcium-phosphate-based material distributed upon at least a portion of the modified section, the modified section and the calcium-phosphate-based material forming at least a portion of a mineralized collagenous scaffold for promoting fixation to bone.

2. The implant of claim 1, wherein the calcium-phosphate-based material is distributed on the porous network and the collagen-based material.

3. The implant of claim 1, wherein the collagen-based material comprises a porous structure.

4. The implant of claim 1, which includes an extended structure contacting the porous network, the extended structure comprising a porous structure including collagen.

5. The implant of claim 1, wherein the calcium-phosphate-based material comprises apatite.

6. The implant of claim 1, wherein the porous network comprises a metallic material preferably at least one of titanium, chromium, and cobalt.

7. The implant of claim 1, wherein the porous network comprises a textured surface.

8. The implant of claim 1, wherein the collagen-based material comprises type I collagen.

9. A method for preparing a mineralized collagenous scaffold to promote bone fixation with an implant, comprising:
exposing a collagen-based material to a porous network of a substrate; and
depositing a calcium-phosphate-based material on at least one of the collagen-based material and the porous network to form the mineralized collagenous scaffold of the implant.

10. The method of claim 9, wherein the step of depositing the collagen-based material to the porous network comprises exposing the at least a portion of the porous network to a collagen-containing mixture, and forming collagen fibrils in the porous network from the mixture.

11. The method of claim 9, wherein the step of exposing the network comprises using an acidic-collagen-containing mixture, and the step of forming collagen fibrils comprises exposing the porous network to a less acidic mixture than the acidic-collagen-containing mixture to form collagen fibrils.

12. The method of claim 11, wherein the step of using the acidic-collagen-containing mixture comprises exposing the porous network to a vacuum before forming the collagen fibrils.

13. The method of claim 9, which includes forming a textured surface on at least a portion of the porous network before attaching the collagen-based material to the porous network.

14. The method of claim 9, which includes applying a bioactive agent to commingle with collagen-based material, preferably by applying a vacuum to drive application of the bioactive agent.

15. The method of claim 9, which includes crosslinking the collagen-based material.
